# EUROPEAN PATENT SPECIFICATION

(11) **EP 1 692 995 B1**
(45) Date of publication and mention of the grant of the patent: **11.11.2015**
(21) Application number: 04820121.4
(22) Date of filing: 25.11.2004
(51) Int. Cl.: A61B 1/06, A61B 1/07, A61B 1/12, A61B 1/00

(54) **ENDOSCOPE**
ENDOSKOP
ENDOSCOPE

(30) Priority: 10.12.2003 JP 2003412613
(43) Date of publication of application: 23.08.2006
(73) Proprietor: Olympus Corporation, Tokyo 151-0072 (JP)
(72) Inventor: TOYAMA, Ryuichi, 1920032 (JP)
(74) Representative: von Hellfeld, Axel
(86) International application number: PCT/JP2004/017462
(87) International publication number: WO 2005/055816

(56) References cited:
- EP-A1- 1 779 763
- JP-A- 2001 258 823
- US-A- 5 575 756
- US-A- 5 871 440

## Description

### TECHNICAL FIELD

The present invention relates to an endoscope, particularly an endoscope having a characteristic distal end structure of its insertion unit.

### BACKGROUND ART

Conventionally, an endoscope has been widely used, for example, in a medical field. The endoscope, for example, can provide observation of organs within a body cavity by inserting an elongated insertion unit into the body cavity and various treatments using a treatment instrument inserted into a treatment instrument insertion channel as necessary. A bendable portion is formed at the distal end of the insertion unit, and the observation direction of an observation window at the distal end can be changed by operating an operation unit of the endoscope.

The conventional endoscope has a viewing angle of, for example, 140 degrees, and an operator observes a body cavity with an observation image at the viewing angle; when the operator wishes to observe a part outside the field of view during the observation of the body cavity, the part outside the field of view can be observed by bending the bendable portion. The endoscope having the viewing angle has two illumination windows at the distal end of the insertion unit, and illumination through the two illumination windows has been sufficient for the viewing angle.

On the other hand, in order to maximize the range of observation, an endoscope with a wider range of the viewing angle (see patent document 1, for example) has been proposed. At the distal end of the insertion unit, plural illumination windows illuminate the object in order to secure the wide field of view and to prevent an observation image displayed on a monitor from losing the quantity of light at a surrounding area thereof.

Also, the endoscope has a variety of functions, especially a function to clean the observation window with a water feeding nozzle, wherein in general, the water feeding nozzle is provided to project a portion thereof from the surface of the distal end.

Patent Document 1: Japanese Patent Application Laid-Open No. H04-102432 (FIG. 3)

JP 2001-258823 discloses an endoscope comprising an observation window, which is arranged closer to one side from a central position of an insert part at a tip of the insert part.

US 5871 440 discloses an endoscope comprising an insertion section and an observation lens positioned on the forward most tip of the rounded distal portion.

### DISCLOSURE OF INVENTION

### PROBLEM TO THE SOLVED BY THE INVENTION

The invention is as defined in the appended claims. When the viewing angle is widened, illumination which illuminates the wider range of area is necessary, and therefore three or more illuminating units are preferably used. However, if three or more illuminating units are mounted, the illumination light hits the water feeding nozzle, thereby giving a tendency to cause flare in the observation image due to the reflection light from the surface of the water feeding nozzle and giving a difficulty in obtaining the clear observation image. In the endoscope with the wide viewing angle relating to the above-described proposal, no consideration had been made as to a possibility of failing to obtain a clear observation image.

Here, it is an object of the present invention to provide an endoscope, with which no flare is generated as the illumination light hits the feed nozzle, and which is capable of obtaining the clearer observation image as a result, even if the endoscope has a wide viewing angle.

### MEANS FOR SOLVING PROBLEM

An endoscope according to the present invention has an insertion unit and is used for an endoscope apparatus which displays an observation image obtained by an imaging device. The endoscope includes an observation window provided on an end surface of a distal end portion of the insertion unit to introduce a reflecting light from an object to the imaging device; a plurality of illuminating units, for emitting illumination light for illuminating the object, provided around the observation window at the end surface of the distal end portion; and a water feeding nozzle, for feeding liquid to a surface of the observation window, provided on the end surface of the distal end portion of the insertion unit. An axis substantially positioned at a center of an illuminating range of the illuminating unit is inclined in a direction away from a distal end of an observing direction of an optical axis of an observation optical system including the observation window, with respect to the optical axis of the observation optical system, so that the illumination light emitted from the plurality of illuminating units does not hit the water feeding nozzle.

An endoscope according to the present invention has an insertion unit and is used for an endoscope apparatus which displays an observation image obtained by an imaging device. The endoscope includes an observation window provided on an end surface of a distal end portion of the insertion unit to introduce a reflecting light from an object to the imaging device; a plurality of illuminating units, for emitting illumination light for illuminating the object, provided around the observation window at the end surface of the distal end portion; and a water feeding nozzle, for feeding liquid to a surface of the observation window, provided on the end surface of the distal end portion. The end surface of the distal end portion has a flat top and a slope which inclines while extending backward from the top, the observation window and the water feeding nozzle are provided at the top, and the plurality of illuminating units are provided along an inclination of the slope.

In the endoscope according to the present invention, the optical axis of the observation optical system is in a same direction as an axis in a longitudinal direction of a channel of the water feeding nozzle.

In the endoscope according to the present invention, the axis substantially positioned at a center portion of an illuminating range by the plurality illuminating units inclines in a direction away from a distal end, toward the object, of a direction of a normal line of a flat surface of the top with respect to the normal line.

In the endoscope according to the present invention, the number of the illuminating units is at least three.

### EFFECT OF THE INVENTION

Even if the endoscope has a wide viewing angle and the illumination light hits the water feeding nozzle, no flare is generated, thereby resulting in providing an effect of obtaining the clear observation image.

### BRIEF DESCRIPTION OF DRAWINGS

FIG. 1 is an explanatory drawing schematically illustrating an endoscope apparatus of an embodiment according to the present invention;
FIG. 2 is an elevational view of a cylindrical distal end portion viewed from the end side thereof;
FIG. 3 is a cross-sectional view of the distal end portion taken along line P-P of FIG. 2;
FIG. 4 is an elevational view of the distal end portion viewed from the end side thereof, illustrating the shape of the distal end portion in view of reduction in the diameter of an insertion unit;
FIG. 5 is a view illustrating an arrangement and direction of each optical fiber bundle, a light guide having three optical fiber bundles being formed on a table;
FIG. 6 is a cross-sectional view of the light guide having the three optical fiber bundles, taken along line Q-Q of FIG. 5;
FIG. 7 is a view illustrating an arrangement and direction of each optical fiber bundle, a light guide having three optical fiber bundles being formed on a table; and
FIG. 8 is a cross-sectional view of the distal end portion with a masking film formed around an imaging unit.

### EXPLANATIONS OF LETTERS OR NUMERALS

- 1: endoscope
- 2: operation unit
- 3: insertion unit
- 7: monitor
- 10: distal end portion
- 22: observation window
- 23a, 23b, 23c: illumination window
- 31: distal end rigid portion
- 32d: imaging device
- 51a, 51b, 51c: optical fiber bundle
- 61: masking film

### BEST MODE(S) FOR CARRYING OUT THE INVENTION

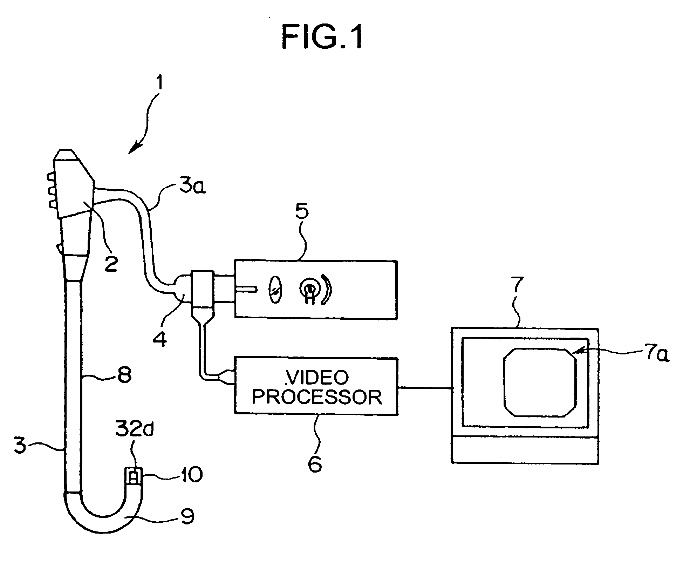

Initially, according to FIG. 1, the configuration of the endoscope apparatus relating to an embodiment of the present invention will be explained. FIG. 1 is an explanatory drawing schematically illustrating the endoscope apparatus according to the embodiment of the present invention. As shown in FIG. 1, this endoscope apparatus includes an endoscope 1 which has a function to obtain an image of a body cavity, a light source 5 which introduces illumination light for obtaining the image into the endoscope 1, a video processor 6 which performs a predetermined image processing on an image signal transmitted from the endoscope 1 and generates an observation image corresponding to the image signal, and a monitor 7 which displays the observation image generated by the video processor 6.

The endoscope 1 includes an operating unit 2 which performs bending operation and controls a channel system, an insertion unit 3 whose proximal end is connected to the operating unit 2 and which is inserted into the body cavity, and a universal cable 3a which is extended out from the operating unit 2 and has a connector portion 4 at its end. The connector portion 4 is connected to the light source 5 and the video processor 6 via a predetermined connector. The video processor 6 is connected to the monitor 7. The insertion unit 3 is provided with a flexible tube 8, a bendable portion 9 provided at the distal end side of the tube 8, and a distal end portion 10 provided at the distal end side of the bendable portion 9. The distal end portion 10 includes an imaging device 32d for obtaining an image of a region within the body cavity.

The image signal of the region within the body cavity obtained by the imaging device 32d positioned in the distal end portion 10 is transmitted to the video processor 6 via the universal cable 3a. The vide processor 6 includes a signal process circuit (not shown in the figures) which processes the transmitted image signal, and based on the processed signal, displays the obtained observation image 7a of the region on the display screen of the monitor 7, which is a display unit connected to the video processor 6.

The operating unit 2 has an operating knob (not shown in the figures) for remotely bending the bendable portion 9. Operating the operating knob generates tensile and relaxation effects of an operating wire (not shown in the figures) inserted in the insertion unit 3, and as a result the bendable portion 9 can be bent in four directions.

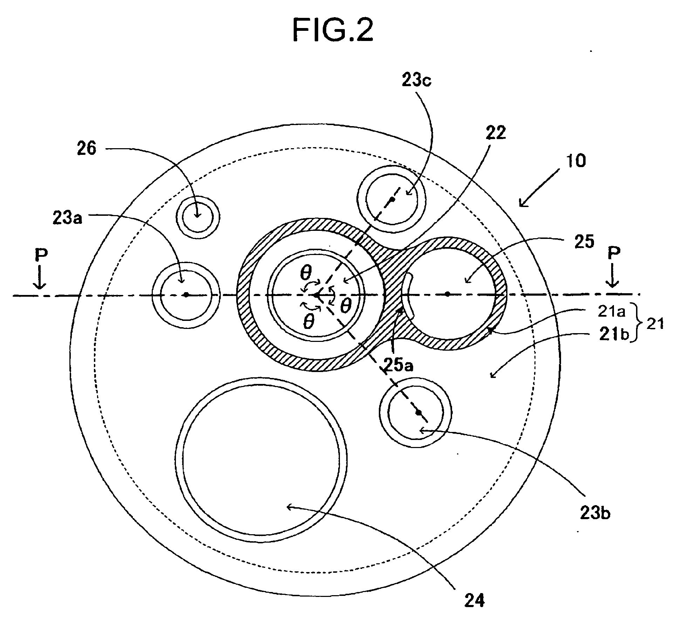

FIG. 2 is an elevational view of the cylindrical distal end portion 10 viewed from its distal end portion. That is, at the end surface 21 of the distal end portion 10, there are one observation window 22, three illumination windows 23a, 23b, and 23c (hereinafter three illumination windows are sometimes collectively represented by 23), an treatment instrument opening 24, a water feeding nozzle 25 which drains for cleaning a surface of a lens functioning as an optical member provided at the observation window 22, and a forward water feeding nozzle 26 which cleans blood, mucus, and the like at an affected part of a subject or the like.

As shown in FIG. 2, on the end surface 21 of the distal end portion 10, three illumination windows 23, when viewed as facing the end surface 21, are positioned around an optical axis of the observation window 22 with a predetermined angle θ, for example, 120 degrees intervals. Furthermore, the treatment instrument opening 24, the water feeding nozzle 25, and the forward water feeding nozzle 26 are positioned around the optical axis of the lens positioned in the observation window 22 and between the respective pairs of the illumination windows. Concretely, the treatment instrument opening 24 is positioned between the illumination window 23a and the illumination window 23b; the water feeding nozzle 25 is positioned between the illumination window 23b and the illumination window 23c; and the forward water feeding nozzle 26 is positioned between the illumination window 23c and the illumination window 23a. That is, three windows corresponding to, for example, the treatment instrument opening 24 are arranged between the respective pairs of the illumination windows 23 in order.

The end surface 21 is configured with a flat top 21a at a peak thereof and a tapered slope 21b which inclines from the top 21a so that a bottom thereof extends backward relative to the end surface 21, i.e., toward a proximal end of the insertion unit 3. The above-descried observation window 22 and the water feeding nozzle 25 are on the top 21a. In this case, the optical axis of a lens to be provided in the observation window 22 is substantially identical to an axis in a direction of a normal line of the flat surface of the top 21a and in a longitudinal direction of the channel of the water feeding nozzle 25. On the other hand, the above-described three illumination windows 23 are positioned at the tapered slope 21b along the inclination thereof, respectively. In this case, an axis which is substantially positioned in almost center of an illuminating area of the respective illumination light emitted from the three illumination windows 23 inclines in a direction to move apart from a point ahead in an observation direction of the optical axis, i.e., a point at the object side, with respect to the longitudinal direction of the channel of the water feeding nozzle 25 and the optical axis of the lens provided in the observation window 22. Also, the treatment instrument opening 24 and the forward water feeding nozzle 26 are positioned at the slope 21b so that the longitudinal direction of the respective channels thereof is substantially identical to the direction of the optical axis of the lens provided in the observation window 22.

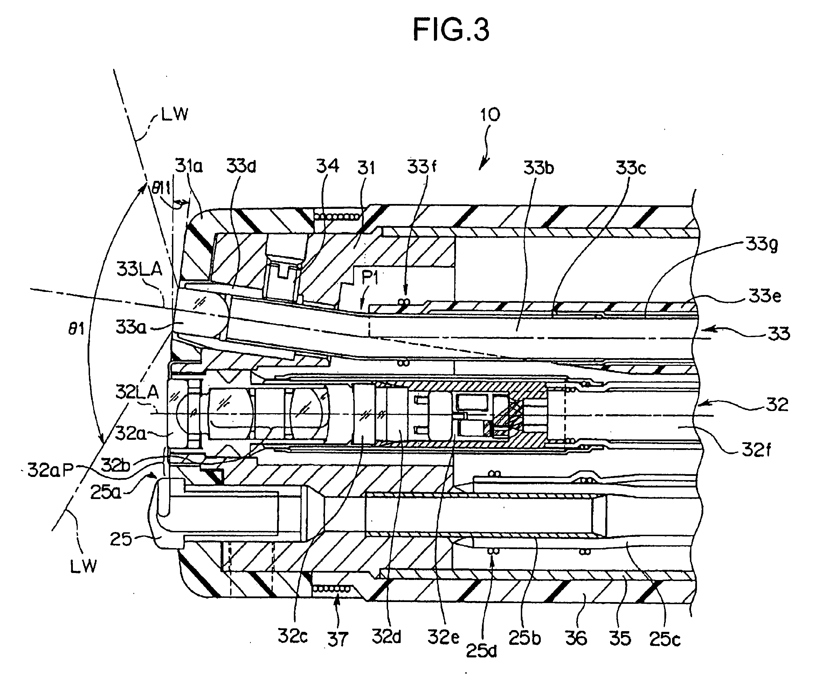

FIG. 3 is a cross-section view of the distal end portion 10 taken along line P-P of FIG. 2. Also, a distal end rigid portion 31 is positioned in the distal end portion 10, and has a space capable of accommodating light guides corresponding respectively to the three illumination windows 23, the imaging unit 32 corresponding to the observation window 22, and the like. A cap 31a is placed so as to cover the end of the distal end rigid portion 31. The imaging unit 32 is inserted into and fixed to the distal end rigid portion 31 so that the observation lens 32a positioned at the end of the imaging unit 32 is arranged in the observation window 22 of the distal end portion 10. The imaging unit 32 includes an observation window lens 32a, an observation optical system 32b of plural lens positioned at a proximal end of the observation window lens 32a, a cover glass 32c positioned at the proximal end of the observation optical system 32b, and the imaging device 32d functioning as a solid imaging device such as CCD positioned at the proximal end of the cover glass 32c. The imaging unit 32 further includes a substrate 32e mounting various circuits thereon and connected to the imaging device 32d. Furthermore, a signal cable 32f is connected to the substrate 32e. The signal cable 32f passes through the insertion unit 3 and is connected to the video processor 6. The imaging unit 32 is fixed to the distal end rigid portion 31 by a screw and filler such as silicon which is not shown in the figure.

A light guide unit 33 is comprised of the illumination window lens 33a and an optical fiber bundle 33b functioning as a light guide. The end of the optical fiber bundle 33b is fixed within a metal pipe 33c by adhesive agent, for example. The end of the optical fiber bundle 33b and the illumination window lens 33a are inserted into and fixed to a frame 33d. The light guide unit 33 is fixed to the distal end rigid portion 31 with a fixing screw 34. The proximal end of the metal pipe 33c and the optical fiber bundle 33b are covered by the flexible tube 33g, and further a portion of the metal pipe 33c and the tube 33g are covered by a covering tube 33e. The covering tube 33e is fixed to the metal pipe 33c with a bobbin winding 33f. The metal pipe 33c, as shown in FIG. 3, is bent at a predetermined position P1 in the middle, and as a result the optical fiber bundle 33b is bent along the shape of bending of the metal pipe 33c as well. Also, as described above, the three illumination windows 23 are provided along the inclination of the slope 21b. Accordingly, the axis 33LA which is substantially positioned in the center of the illuminating area of the illuminating unit such as the illumination window lens 33a which emits the illumination light (hereinafter for the sake of convenience in explaining, the axis is referred to as illumination center axis) is not parallel to an optical axis 32LA of the observation optical system such as the observation window lens of the imaging unit 32. Especially, the illumination center axis 33LA is inclined with respct to the optical axis 32LA in a direction that the end of the illumination center axis 33LA is away from a distal end of the observation direction of the optical axis 32LA of the imaging unit 32 and the illumination center axis 33LA. Like the illumination center axis 33LA, the illumination center axes of the light guide units corresponding respectively to other illumination windows 23b, 23c is inclined with respect to the optical axis 32LA in a direction that each end of the axes is away from a distal end of the observation direction of the optical axis 32LA of the imaging unit 32.

In addition, the illumination center axis is an axis substantially positioned in the center of the illuminating area of the illumination light emitted by an illumination unit including the later described LED; here, the illumination center axis is the optical axis of the optical system of, for example, the illumination window 23a included in the illumination unit.

The distal end of the water feeding nozzle 25 is provided with an opening 25a. The opening 25a is designed so that water ejected from the water feeding nozzle 25 is fed in a direction substantially parallel to a flat surface perpendicular to the optical axis 32LA of the imaging unit 32 and in a direction to pass through the surface of the observation window lens 32a at the observation window 22 and the surface of the illumination lens 33a at the illumination window 23a. The proximal end of the water feeding nozzle 25 is pipe-shaped and is connected to a water feeding tube 25c via a connecting tube 25b. Accordingly, the connecting tube 25b and the water feeding tube 25c constitute a water feeding tube channel. The water feeding tube 25c is fixed to the connecting tube 25b by the bobbin winding 25d.

The proximal end of the distal end rigid portion 31 is fixed to one portion of a curved top coma 35. The proximal end of the distal end rigid portion 31 and the curved top coma 35 are covered by the covering tube 36. The covering tube 36 is fixed to the distal end rigid portion 31 with the bobbin winding 37.

Next, the positional relationship between the observation window 22 at the distal end portion 10 and one illumination window of the three illumination windows 23 is explained next. As shown in FIG. 2, a shape of the distal end portion 10 in cross section in the direction perpendicular to the optical axis 32LA of the observation optical system is circular. The observation window 22 is positioned at the top 21 of the end surface 21 of the distal end portion 10 so as to arrange the central position of the observation window 22 comes to a position off from the central position of the circle. The respective three illumination windows 23 are positioned with a predetermined distance therebetween around the observation window 22 at the slope 21b of the end surface 21 of the distal end portion 10.

Furthermore, within the insertion unit 3, in addition to the imaging unit 32, the light guide of the optical fiber bundle corresponding to the three illumination windows 23 and the channel as a built-in component corresponding to the treatment instrument opening 24, the water feeding nozzle 25, and the forward water feeding nozzle 26 are inserted. As such, in addition to the imaging unit 32, six built-in components are positioned within the distal end portion 10, and the diameter of the distal end portion 10 needs to be kept from becoming large. Here, as shown in FIG. 2, the treatment instrument opening 24, the water feeding nozzle 25, and the forward water feeding nozzle 26 of the end of three built-in components are arranged in turn among the three illumination windows 23, thereby emitting the illumination light with excellent balance and preventing the diameter of the distal end portion 10 from becoming large in the endoscope with a wide viewing angle.

Furthermore, as shown in FIG. 2, on the end surface 21 of the distal end portion 10 of the insertion unit 3, the water feeding nozzle 25 at the distal end of the water feeding channel and the illumination window 23a are shown on almost straight line P-P having the observation window 22 therebetween. This arrangement is to remove fouling of at least one illumination window lens 33a together with the observation window lens 32a by water ejected from the opening portion 25a of the water feeding nozzle 25 even if fouling adheres to the end surface 21 of the distal end portion 10 of the insertion unit 3. Accordingly, while observing with the endoscope, minimum requirement with respect to the amount of illumination is constantly secured, thereby obtaining an excellent observability. Especially, in FIG. 2, the center of the water feeding nozzle 25 and the center of the illumination window 23a are point symmetrical to the center of the observation window 22.

The imaging device 32d transmits the image signal to the video processor 6 by light obtained through the observation window 22; however, the video processor 6 performs the image processing on the image signal and generates the data of the observation image 7a of substantially rectangular in its shape. The substantially rectangular observation image 7a, as shown in FIG. 1, has four corners trimmed, i.e., providing electronic masking, and shows its octangle observation image on the monitor 7. Also, here, the optical system of the imaging unit 32 is designed so that the water feeding nozzle 25 does not go in the observation field of vision of the imaging unit 32.

Next, the illuminating area of the illumination light from the illumination window 23 is explained. As shown in FIG. 3, the illumination light from the illumination window lens 33a of the light guide unit 33 is emitted from the illumination window lens 33a with the illuminating area as shown in one dashed line LW. This illuminating area LW is an area defined by the illumination angle θ1 taking the illumination center axis 33LA of the illumination window lens 33a as a center. The illuminating area LW is determined by designing the optical system of the illumination window lens 33a of the light guide unit 33. When the water feeding nozzle 25 exists in the illuminating area LW, reflection such as irregular reflection of the illumination light is affected on the surface of the water feeding nozzle 25.

Here, in order to avoid causing such reflection and to prevent the water feeding nozzle 25 from going in the illuminating area LW defined by the illumination angle θ1 of the illumination light from the illumination window lens 33a, the illumination center axis 33LA of the illuminating unit including the illumination window lens 33a is inclined with respect to the optical axis 32LA in a direction that the end of the illumination center axis 33LA is away from a distal end of the observation direction of the optical axis 32LA of the observation optical system including the observation window 22. As a result, the water feeding nozzle 25 is not included in the illuminating area LW, and the reflection of the illumination light does not occur on the surface of the water feeding nozzle 25, thereby preventing flare in the observation image as reflecting light hits the observation window 22.

Especially, as shown in FIG. 3, the illumination center axis 33LA of the illumination window lens 33a is inclined to the optical axis 32LA of the imaging unit 32. At this time, the illuminating area LW of the illumination light emitted from the illumination window lens 33a having the inclination angle θ11 relative to the surface of the observation window lens 32a is an area that does not cover the water feeding nozzle 25. As such, the illumination center axis 33LA is inclined relative to the optical axis 32LA, and the illuminating area LW is designed so that the illumination does not hit the water feeding nozzle 25, thereby not being an obstacle to cleaning the illumination lens 33a by water ejected from the water feeding nozzle 25 to prevent the illumination of the illumination light to the water feeding nozzle 25 and further expanding the illuminating area LW more than when forming a blocking wall between the illumination window 23a and the observation window 22 to cut off the illumination light for the illumination light not to hit the water feeding nozzle 25.

Similarly, the illumination light from the respective illumination window lenses provided in the illumination windows 23b, 23c is designed not to hit the water feeding nozzle 25 as well. That is, in order for the water feeding nozzle 25 not to be in the illuminating area defined by the illumination angles θ2 and θ3 (not shown in the figures) of the respective illumination light from the respective illumination window lenses provided in the illumination windows 23b, 23c, the respective illumination center axes by the respective illuminating units including the respective illumination window lenses provided in the illumination windows 23b, 23c is inclined with respect to the optical axis 32LA of the observation optical system including the observation window lens 32a in the direction to move apart from a distal end of the observation direction. At this time, the illuminating area of the respective illumination light emitted from the illumination window lens of the illumination window 23b and the illumination lens of the illumination window 23c having the inclination angles θ21 and θ31 (not shown in the figures) relative to the surface of the observation window lens 32a respectively is an area that does not cover the water feeding nozzle 25.

Accordingly, there is no chance that all illumination light of the three illumination windows 23 hits the water feeding nozzle 25 and the reflection light goes in the observation window 22, thereby obtaining a clear observation image without flare, and the distance between the illumination window 23 and the water feeding nozzle 25 does not need to be reserved excessively so as to avoid flare, thereby enabling to minimize the size of the distal end portion 10.

Also, in the above-explanation, an example employing the light guide unit including the illumination window unit and the like was explained as the illumination means; however, a light emitting diode (LED) can be used on the end surface 21 of the distal end portion 10 as a light emitting element. At that time, plural LEDs can be arranged along the inclination of the slope 21b of the end surface 21, and the illumination center axis of the respective LEDs is positioned in the direction to move apart from a distal end of the observation direction of the optical axis of the observation optical system and is inclined with respect to the optical axis of the observation optical system.

Also, in the above-example, the example with three illuminating units provided therein was explained because this example provides advantages in illuminating sufficient amount of light to the object even with three illuminating units and in addition reducing the outside diameter of the distal end comparing to the case when four or more illuminating units are provided.

On the other hand, as ejecting.water, i.e., liquid, from the opening portion 25a of the water feeding nozzle 25, an object such as fouling adhered on the surface of the observation window lens 32a provided in the observation window 22 needs to be removed. This is because when the insertion unit 3 of the endoscope 1 is inserted in the body cavity, the object such as fouling adheres on the surface of the observation window lens 32a.

However, even though JP-A No. H04-102432 (KOKAI) discloses the endoscope having a wide viewing angle, water from the water feeding nozzle disclosed in FIG. 2 of the same publication often is unable to remove fouling or the like adhered on the lens surface of the observation window sufficiently because the water hits from the lateral direction of a convex spherical surface.

Here, in the present embodiment, the observation window 22 and the illumination window 23a are provided at the top 21a of the end surface 21 and the surrounding surface of the observation window 22 has a plane shape as described above. Concretely, as shown in FIG. 3, at the end surface 21 of the distal end portion 10, an area 32aP from the opening 25a of the water feeding nozzle 25 to the observation window lens 32a is formed in a flat surface. Accordingly, at least at the end surface 21 of the distal end portion 10, the area 32aP from the opening 25a of the water feeding nozzle 25 to the observation window lens 32a is formed in a flat surface, and the water from the opening 25a of the water feeding nozzle 25 flows along the flat surface and is delivered appropriately relative to the surface of the observation window lens 32a which is an objective lens. In other words, at the end surface 21 of the cover 31a of the distal end portion 10, the flat surface area 32aP substantially identical to the surface of the observation window lens 32a is provided between the opening 25a of the water feeding nozzle 25 and the observation window 22. As a result, the water from the opening 25a of the water feeding nozzle 25 hits appropriately on the surface of the observation window lens 32a functioning as an objective lens along the flat surface, which improves the cleaning effect on the surface of the observation window lens 32a.

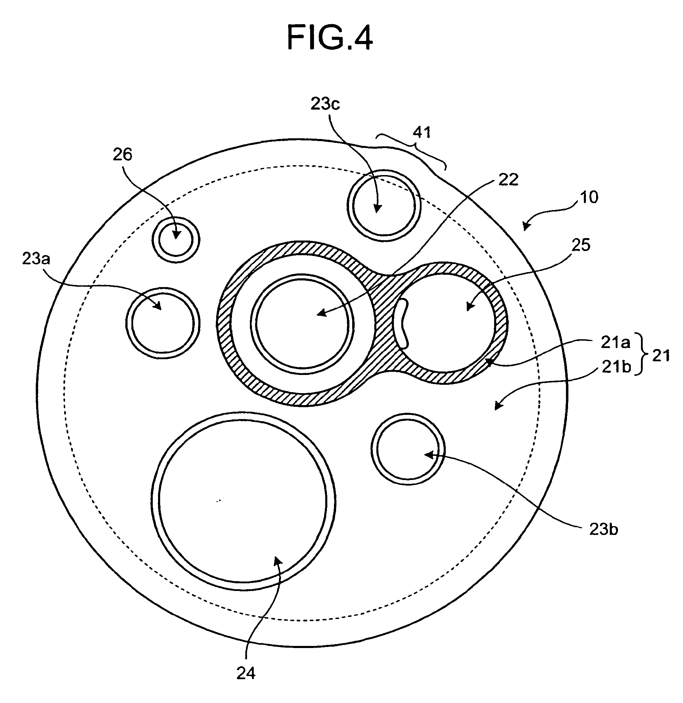

Also, the distal end portion 10 of the endoscope 1 is preferably narrower in consideration of insertability and pain to the patients. FIG. 4 is an elevational view viewed from the end side of the distal end portion 10 for the explanation of the shape of the distal end portion 10 in consideration of reduction in the diameter of the distal end portion 10. FIG. 4 uses the identical reference numbers as in FIG. 2 for the identical components, the explanation of which is not provided.

The observation window 22, three illumination windows 23, and the treatment instrument opening 24, and the water feeding nozzle 25 are provided at the end surface 21 of the distal end portion 10. However, as shown in FIG. 4, the cross section shape of the distal end portion 10 at the surface perpendicular to the axis of the distal end portion 10 is not a simple circular shape but is a circular shape deformed to correspond to a part of components to form a projection 41.

As shown in FIG. 4, at the distal end portion 10, the observation window 22, the two illumination windows 23a, 23b, the treatment instrument opening 24, the water feeding nozzle 25, and the forward water feeding nozzle 26 can be arranged in a predetermined circle shown in a dashed line which is representing the respective window and the storing area of the respective built-in component; however, one illumination window 23c cannot fit in the predetermined circle. Then, a shape viewed from the front surface of the end surface of the distal end portion 10 corresponding to the illustration window 23c is designed to be shaped with the projection 41 at one portion thereof.

Furthermore, at this time, the built-in component such as the optical fiber bundle corresponding to the illumination window 23c may not fit in the dashed line circle. In such a case, the cross section shape of the distal end portion 10 from the end surface 21 of the distal end portion 10 to a position slightly apart toward the proximal end, i.e., a position where the built-in component such as the optical fiber corresponding to the illumination 23c fits in the predetermined circle in the dashed line, has the projection at one portion thereof.

By making this cross section shape, the diameter of the distal end portion 10 can be reduced, thereby improving the insertability of the insertion unit and relieving the pain of the patients.

The above-explanation illustrates, in FIG. 4, the case that only the illumination window 23c cannot fit in the predetermined circle; however, similarly, the shape of the distal end portion 10 viewed from the front surface of the end surface 21 of the distal end portion 10 can have the projection when other components such as the illumination window 23a, 23b and the water feeding nozzle 25 do not fit in the circle,

Next, a method of forming the light guide in the manufacturing process of the above-described insertion unit 3 will be explained. As explained in FIGS. 2 and 3, three optical fiber bundles of the light guide unit 33 are provided in correspondence to the illumination windows 23. Furthermore, the respective optical fiber bundle is bent in the middle adjacent to the illumination window 23. As a result, as explained above, the illumination center axes of the respective illumination windows emitting the illumination light corresponding respectively to the optical fiber bundles bent in the middle are not parallel to the optical axis 32LA of the imaging unit 32.

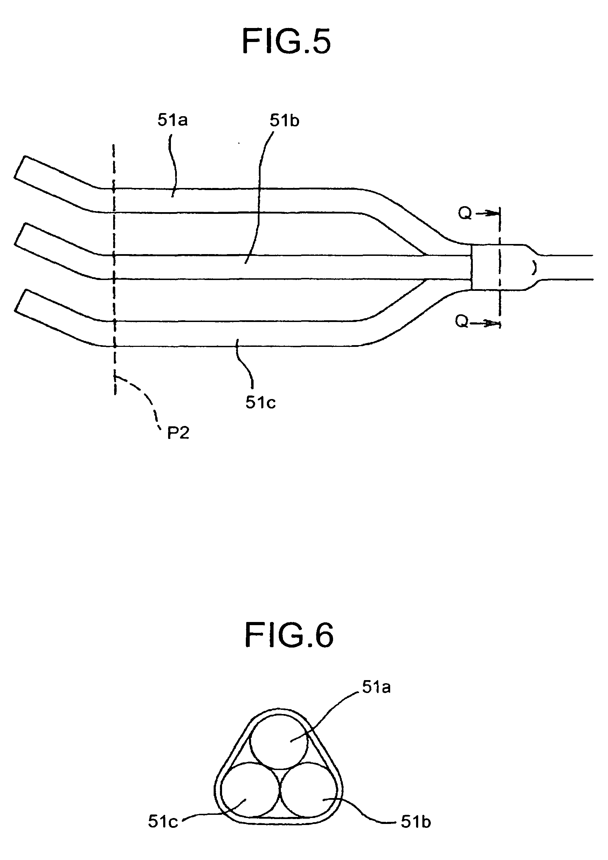

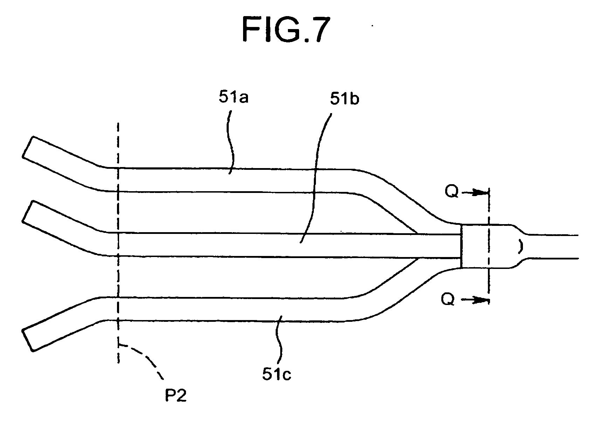

The three optical fiber bundles with the bent ends are bundled into one within the insertion unit 3 and are connected to the light source 5. FIGS. 5 and 7 are schematic views illustrating the arrangement and direction of the respective optical fibers in the cases that a light guide having three optical fiber bundles is formed on the plane table. FIG. 6 is a cross-section view of the light guide having three optical fiber bundles taken along the line Q-Q of FIG. 5.

The end side of each optical fiber bundle is formed by binding the same by the adhesive agent. Here, when the end of the respective optical fiber bundle is formed by the adhesive agent, as shown in FIG. 5, on a two dimensional flat surface, i.e., a plan surface table, the end of the respective optical fiber bundle can be formed to be directed in the same direction. For example, as shown in FIG. 5, the bundle of three optical fiber bundles 51a, 51b, and 51c is arranged in parallel on the plane table, and the respective distal ends, i.e., a portion at a left side shown by a dashed line P2 in FIG. 5, is bent in the same direction, and the adhesive agent is used to complete the forming. Then, the three optical fiber bundles 51a, 51b, 51c (hereinafter, three optical fiber bundles are often referenced as 51) at its proximal end are such that three optical fibers are packed in the closest contact as shown in the cross section view of FIG. 6. Packaging in the closest contact in the cross section provides advantages in better marinating the shape and saving the space.

However, if the end side portion of the three optical fiber bundles 51 are formed by bending in the same direction as shown in FIG. 5, one optical fiber bundle 51c out of three is twisted more than the other two optical fiber bundles as being inserted in the distal end rigid portion 31 of the insertion unit 3, when assembling the three optical fiber bundles 51 in the distal end rigid portion 31 of the insertion unit 3.

Then, as shown in FIG. 7, when only one optical fiber bundle 51c of three optical fiber bundles is placed on the plane table and is formed, the end thereof is bent in a different direction from the other two optical fiber bundles 51a, 51b. The remaining two optical fiber bundles 51a, 51b are directed in the same direction, and only one optical fiber bundle 51c is formed as being directed in a different, i.e., opposite, direction. Structuring as such would eliminate the chance of one optical fiber bundle of three being more twisted when being assembled in the distal end portion 10.

Furthermore, after manufacturing the endoscope 1, for example, when inspecting the same, there is a chance where only the imaging unit 32 needs to be replaced because of failure. However, as described above, when the imaging unit 32 is fixed in the distal end rigid portion 31 by filling the filler such as silicon rubber in the distal end rigid portion 31, the imaging unit 32 cannot be removed alone from the distal end rigid portion 31 practically.

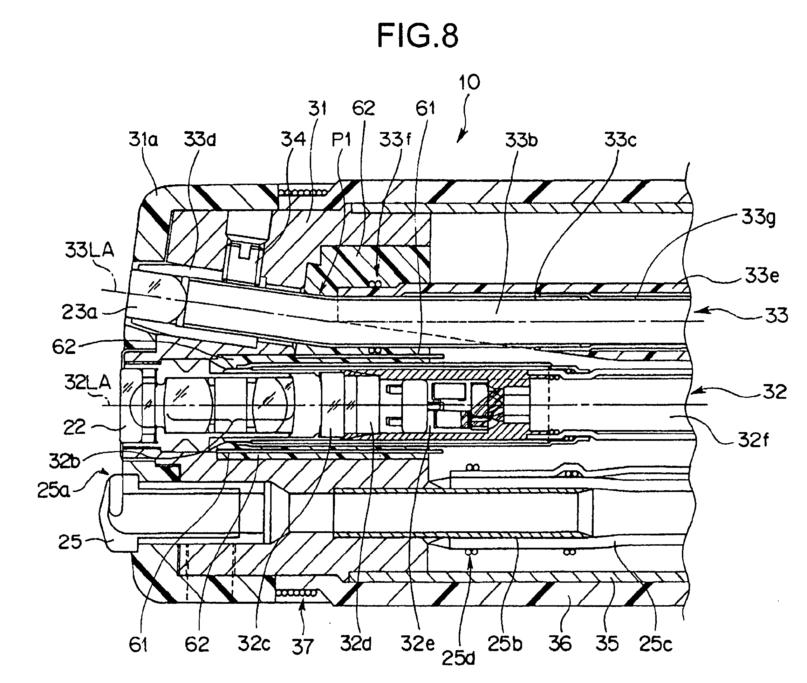

Then, as shown in FIG. 8, at the distal end portion 10 of the insertion unit 3 of the endoscope 1, the filler 62 is filled under the condition that the masking film made of film material is positioned around the imaging unit 32. FIG. 8 is a cross-section view of the distal end portion 10 with the masking film 61 positioned around the imaging unit 32. In FIG. 8, the components same as the ones in FIG. 3 have identical reference numbers and the explanation is not provided here.

As shown in FIG. 8, the masking film 61 is positioned around the imaging unit 32, and especially within the distal end rigid portion 31, the imaging unit 32 and the built-in component therearound such as the light guide unit 33 are not fixed via the filler 62 only. That is, within the distal end rigid portion 31, the imaging unit 32 and the built-in component therearound are fixed to the distal end rigid portion 31; however, the imaging unit 32 and the built-in component therearound is designed such that the masking film 61 is positioned around the imaging unit 32 so as to be fixed via the masking film 61 mutually. The masking film 61 can be a single layer around the imaging unit 32 or can be double layer or more to be rolled.

As such, within the distal end rigid portion 31, the masking film 61 is positioned around the imaging unit 32, and if only the imaging unit 32 is attempted to be pulled out from the distal end rigid portion 31, only the imaging unit 32 wrapped inside the masking film 61 can be pulled from the distal end rigid portion 31 together with the masking film 61 or as being peeled from the masking film 61 because of the existence of the masking film 61.

Accordingly, even if the filler such as silicon is filled in the distal end rigid portion 31 to fix the imaging unit 32 at the distal end rigid portion 31, only the imaging unit 32 can be taken from the distal end rigid portion 31.

As such, according to the embodiments of the present invention, even if the endoscope has three illumination windows, light from the three windows is distributed with good balance, thereby realizing the endoscope with excellent observability.

### INDUSTRIAL APPLICABILITY

As such, the endoscope according to the present invention is desirable for process of obtaining a clear image of an aimed object in the body cavity and desirable especially for an endoscope apparatus for displaying the image of body cavity of a subject to observe inside of the body cavity of the subject.

The embodiements, aspects and examples which do not fall within the scope of the Claims are provided for illustrative purpose only and do not form part of the present invention.

The invention is defined in the claims as follows.

## Claims

1. An endoscope (1) comprising:
an insertion unit (3) having an end surface (21) and insertable into a body cavity, the end surface (21) having a flat top (21a) and a slope (21b) expanding from the flat top (21a);
an imaging device (32) obtaining an image of a region within the body cavity;
a plurality of illuminating units (23a, 23b, 23c) emitting illumination light to the region, emitting end surfaces of the illuminating units (23a, 23b, 23c) being arranged along the slope (21b);
an observation optical system (32) including an observation window (22) having an optical axis (32LA) provided on the flat top (21a) of the end surface (21) and guiding a light reflected from the region to the imaging device (32) through the observation window (22);and
a water feeding nozzle (25), provided on the flat top (21a) of the end surface (21) and feeding liquid to a surface of the observation window (22) and having an opening (25a) to eject the liquid therethrough in a direction substantially parallel to the flat top (21a), **characterized in that**
a flat surface area (32aP) substantially identical to the surface of the observation window (22) is provided between the opening (25a) of the water feeding nozzle (25) and the observation window (2), wherein the opening (25a) of the water feeding nozzle (25) is located on a position projecting from the observation window (22) with respect to the plurality of illuminating units (23a, 23b, 23c) on the slope (21b) of the end surface (21) and the liquid from the opening (25a) of the water feeding nozzle (25) flows along the flat surface area (32aP) to perform a cleaning effect on the surface of the observation window (22), wherein
the flat top (21a) extends perpendicular to the optical axis (32LA) of the observation optical system (32).

2. The endoscope (1) according to claim 1, wherein
the optical axis (32LA) of the observation optical system (32) is in a same direction as an axis in a longitudinal direction of a channel of the water feeding nozzle (25).

3. The endoscope (1) according to claim 1, wherein
an axis (33LA) substantially positioned at a center portion of an illuminating range by the plurality illuminating units (23a, 23b, 23c) inclines in a direction away from a distal end, toward the body cavity, of a direction of a normal line of a flat surface of the flat top (21a) with respect to the normal line.

4. The endoscope (1) according to any one of claims 1 to 3, wherein the number of the illuminating units is at least three.

## Patentansprüche

1. Endoskop (1) aufweisend:
eine Einführeinheit (3), die eine Endfläche (21) aufweist und in eine Körperhöhle einführbar ist, wobei die Endfläche (21) ein flaches oberes Ende (21a) und eine Neigung (21b) aufweist, die sich von dem flachen oberen Ende (21a) erstreckt;
eine Bildgebungsvorrichtung (32) zum Aufnehmen eines Bildes einer Region innerhalb der Körperhöhle;
eine Vielzahl von Beleuchtungseinheiten (23a, 23b, 23c) zum Abgeben von Beleuchtungslicht zu der Region, wobei abgebende Endflächen der Beleuchtungseinheiten (23a, 23b, 23c) entlang der Neigung (21b) angeordnet sind;
ein optisches Beobachtungssystem (32), das ein an dem flachen oberen Ende (21a) der Endfläche (21) vorgesehenes Beobachtungsfenster (22) und eine optische Achse (32LA) aufweist, und das von der Region reflektiertes Licht durch das Beobachtungsfenster (23) zu der Bildgebungsvorrichtung (32) führt; und
eine Wasserzuführdüse (25), die an dem flachen oberen Ende (21a) der Endfläche (21) vorgesehen ist und Flüssigkeit zu einer Fläche des Beobachtungsfensters (22) führt und mit einer Öffnung (25a) zum Abgeben der Flüssigkeit durch die Öffnung (25a) in eine Richtung im Wesentlichen parallel zu dem flachen oberen Ende (21a), **dadurch gekennzeichnet, dass**
ein flacher Flächenbereich (32aP), der im Wesentlichen identisch zu der Fläche des Beobachtungsfensters (22) ist, zwischen der Öffnung (25a) der Wasserzuführdüse (25) und dem Beobachtungsfenster (22) vorgesehen ist, wobei die Öffnung (25a) der Wasserzuführdüse (25) an einer Position angeordnet ist, die von dem Beobachtungsfenster (22) bezüglich der Vielzahl der Beleuchtungseinheiten (23a, 23b, 23c) an der Neigung (21b) der Endfläche (21) vorsteht und die Flüssigkeit von der Öffnung (25a) der Wasserzuführdüse (25) entlang des flachen Flächenbereichs (32aP) fließt, um einen Reinigungseffekt an der Fläche des Beobachtungsfensters (22) auszuführen, wobei
sich das flache obere Ende (21a) senkrecht zu der optischen Achse (32LA) des optischen Beobachtungssystems (32) erstreckt.

2. Endoskop (1) nach Anspruch 1, bei dem
die optische Achse (32LA) des optischen Beobachtungssystems (32) in derselben Richtung wie eine Achse in Längsrichtung eines Kanals der Wasserzuführdüse (25) verläuft.

3. Endoskop (1) nach Anspruch 1, bei dem
eine Achse (33LA), die im Wesentlichen an einem zentralen Abschnitt eines Beleuchtungsbereichs der Vielzahl der Beleuchtungseinheiten (23a, 23b, 23c) positioniert ist, sich zu der Körperhöhle hin in einer Richtung weg von einem distalen Ende einer Richtung einer Normalen einer flachen Fläche des flachen oberen Endes (21a) bezüglich der Normalen neigt.

4. Endoskop (1) nach einem der Ansprüche 1 bis 3, bei dem
die Anzahl der Beleuchtungseinheiten wenigstens drei ist.

## Revendications

1. Endoscope (1) comprenant :
une unité d'insertion (3) comportant une surface d'extrémité (21) et pouvant être insérée dans une cavité de corps, la surface d'extrémité (21) comportant un sommet plat (21a) et une pente (21b) s'étendant depuis le sommet plat (21a) ;
un dispositif d'imagerie (32) obtenant une image d'une région à l'intérieur de la cavité de corps ;
une pluralité d'unités d'éclairage (23a, 23b, 23c) émettant une lumière d'éclairage vers la région, des surfaces d'extrémité émettrices des unités d'éclairage (23a, 23b, 23c) étant agencées le long de la pente (21b) ;
un système optique d'observation (32) comprenant une fenêtre d'observation (22) comportant un axe optique (32LA) prévu sur le sommet plat (21a) de la surface d'extrémité (21) et guidant une lumière réfléchie depuis la région vers le dispositif d'imagerie (32) à travers la fenêtre d'observation (22) ; et
une buse d'alimentation en eau (25), prévue sur le sommet plat (21a) de la surface d'extrémité (21) et délivrant du liquide vers une surface de la fenêtre d'observation (22) et comportant une ouverture (25a) pour éjecter le liquide à travers celle-ci dans une direction sensiblement parallèle au sommet plat (21a), **caractérisé en ce que**
une zone de surface plate (32aP) sensiblement identique à la surface de la fenêtre d'observation (22) est prévue entre l'ouverture (25a) de la buse d'alimentation en eau (25) et la fenêtre d'observation (22), dans laquelle l'ouverture (25a) de la buse d'alimentation en eau (25) est placée sur une position faisant saillie depuis la fenêtre d'observation (22) par rapport à la pluralité d'unités d'éclairage (23a, 23b, 23c) sur la pente (21b) de la surface d'extrémité (21) et le liquide provenant de l'ouverture (25a) de la buse d'alimentation en eau (25) circule le long de la zone de surface plate (32aP) pour réaliser un effet de nettoyage sur la surface de la fenêtre d'observation (22), dans lequel
le sommet plat (21a) s'étend perpendiculaire à l'axe optique (32LA) du système optique d'observation (32).

2. Endoscope (1) selon la revendication 1, dans lequel
l'axe optique (32LA) du système optique d'observation (32) se trouve dans un sens identique à un axe dans un sens longitudinal d'un canal de la buse d'alimentation en eau (25).

3. Endoscope (1) selon la revendication 1, dans lequel
un axe (33LA) sensiblement positionné au niveau d'une partie centrale d'une plage d'éclairage par la pluralité d'unités d'éclairage (23a, 23b, 23c) s'incline dans une direction éloignée d'une extrémité distale, vers la cavité de corps, dans la direction d'une ligne normale d'une surface plate du sommet plat (21a) par rapport à la ligne normale.

4. Endoscope (1) selon l'une quelconque des revendications 1 à 3, dans lequel le nombre d'unités d'éclairage est d'au moins trois.
